# EUROPÄISCHE PATENTSCHRIFT

(11) **EP 1 366 005 B1**
(45) Veröffentlichungstag und Bekanntmachung des Hinweises auf die Patenterteilung: **23.05.2007**
(21) Anmeldenummer: 02712897.4
(22) Anmeldetag: 06.02.2002
(51) Int. Cl.: C07C 11/167, C07C 7/08, B01D 3/40

(54) **VERFAHREN ZUR GEWINNUNG VON ROH-1,3-BUTADIEN DURCH EXTRAKTIVDESTILLATION AUS EINEM C4-SCHNITT**
METHOD FOR RECOVERING CRUDE 1,3-BUTADIENE BY EXTRACTIVE DISTILLATION FROM A C4 CUT
PROCEDE POUR EXTRAIRE DU 1,3-BUTADIENE BRUT PAR DISTILLATION EXTRACTIVE DANS UNE FRACTION C 4?

(30) Priorität: 08.02.2001 DE 10105660
(43) Veröffentlichungstag der Anmeldung: 03.12.2003
(73) Patentinhaber: BASF Aktiengesellschaft, 67056 Ludwigshafen (DE)
(72) Erfinder: BOHNER, Gerd, 69254 Malsch (DE); KINDLER, Klaus, 67376 Harthausen (DE); PAHL, Melanie, 68167 Mannheim (DE); KAIBEL, Gerd, 68623 Lampertheim (DE)
(74) Vertreter: Isenbruck, Günter
(86) Internationale Anmeldenummer: PCT/EP2002/001219
(87) Internationale Veröffentlichungsnummer: WO 2002/062733

(56) Entgegenhaltungen:
- DE-A- 2 724 365
- DE-A- 10 022 465

## Beschreibung

Die Erfindung betrifft ein Verfahren zur Gewinnung von Roh-1,3-Butadien durch Extraktivdestillation aus einem C₄-Schnitt mit einem selektiven Lösungsmittel sowie eine hierfür geeignete Anlage.

Die Gewinnung von Roh-1,3-Butadien aus einem C₄-Schnitt ist wegen der geringen Unterschiede der Komponenten des C₄-Schnittes in den relativen Flüchtigkeiten ein kompliziertes destillationstechnisches Problem. Daher wird die Auftrennung durch eine sogenannte Extraktivdestillation durchgeführt, das heißt eine Destillation unter Zugabe eines Extraktionsmittels, das einen höheren Siedepunkt als das aufzutrennende Gemisch aufweist und das die Unterschiede in den relativen Flüchtigkeiten der aufzutrennenden Komponenten erhöht. Durch Einsatz geeigneter Extraktionsmittel kann aus dem genannten C₄-Schnitt mittels Extraktivdestillation eine Roh-1,3-Butadienfraktion erhalten werden, die anschließend in Reindestillationskolonnen weitergereinigt wird neben einem Strom, der die weniger löslichen Kohlenwasserstoffe als 1,3-Butadien, insbesondere Butane und Butene enthält, sowie einem Strom, der die leichter löslichen Kohlenwasserstoffe als 1,3-Butadien, insbesondere die Butine sowie gegebenenfalls 1,2-Butadien enthält.

Vorliegend wird als Roh-1,3-Butadien ein Kohlenwasserstoffgemisch bezeichnet, das das Wertprodukt 1,3-Butadien in einem Anteil von mindestens 80 Gew.-%, bevorzugt 90 Gew.-%, besonders bevorzugt 95 Gew.-%, Rest Verunreinigungen, enthält.

Dem gegenüber wird als Rein 1,3-Butadien ein Kohlenwasserstoffgemisch bezeichnet, das das Wertprodukt 1,3-Butadien in einem Anteil von mindestens 99 Gew.-%, bevorzugt 99,5 Gew.-%, besonders bevorzugt 99,7 Gew.-%, Rest Verunreinigungen enthält.

Die DE-A 27 24 365 beschreibt ein Verfahren zur Gewinnung von 1,3-Butadien aus einem C₄-Schnitt wobei zunächst durch Extraktivdestillation ein Roh-1,3-Butadien erhalten wird, das anschließend destillativ zu Rein-1,3-Butadien weiterverarbeitet wird.

Nach dem Verfahren der DE-A 27 24 365 erfolgt die Extraktivdestillation in einer Anlage mit drei Kolonnen, dem sogenannten Hauptwascher, Gegenströmer bzw. Nachwascher. Im Hauptwascher wird der verdampfte C₄-Schnitt im Gegenstrom mit dem Extraktionsmittel, insbesondere N-Methylpyrrolidon, im folgenden abgekürzt als NMP bezeichnet, in Kontakt gebracht. Dabei werden die in NMP besser löslichen Komponenten Propin, Butenin, 1-Butin, 1,2-Butadien, 1,3-Butadien sowie cis-2-Buten absorbiert. Die in NMP schlechter als 1,3-Butadien löslichen Komponenten, insbesondere ein Gemisch aus Butenen und Butanen, werden am Kopf des Hauptwaschers abgezogen. Das Sumpfprodukt des Hauptwaschers wird auf den Kopf der zweiten Kolonne der Extraktivdestillationsanlage, des Gegenströmers, gepumpt. Der Gegenströmer besteht aus einem Ober- und einem Unterteil, die unterschiedliche Aufgaben haben: Das Oberteil stellt verfahrenstechnisch die Verlängerung des Hauptwaschers dar, während das Unterteil dem Nachwascher zuzuordnen ist. Im Oberteil werden die im Lösungsmittel gelösten Restanteile von Butenen ausgestrippt und dem Hauptwascher erneut zugeführt. Am Übergang vom Unterteil zum Oberteil des Gegenströmers wird ein mit 1,3-Butadien angereicherter Strom, der außerdem noch im Lösungsmittel besser als 1,3-Butadien lösliche Komponenten, insbesondere C₃- und C₄-Acetylene, sowie 1,2-Butadien, cis-2-Buten und C₅-Kohlenwasserstoffe enthält, abgezogen. Da somit am Übergang vom Unterteil zum Oberteil des Gegenströmers ein Teil des aufströmenden Dampfes abgezogen wird, muß, um einen entsprechend guten Stoffübergang in der gesamten Kolonne zu gewährleisten, aus hydrodynamischen Gründen das Oberteil des Gegenströmers im Vergleich zum Unterteil mit kleinerem Durchmesser ausgeführt werden. Die hierfür erforderliche Verjüngung ist konstruktionstechnisch schwieriger zu realisieren gegenüber einem Apparat mit konstantem Durchmesser über die gesamte Höhe.

Im Gegenströmersumpf erfolgt eine Vorausgasung der in NMP gelösten Kohlenwasserstoffe; teilentgastes NMP wird zur vollständigen Entgasung in die Ausgaserkolonne gepumpt.

Aus dem am Übergang zwischen Unterteil und Oberteil des Gegenströmers abgezogenen dampfförmigen 1,3-butadienhaltigen Strom werden in einer dritten Kolonne der Extraktivdestillationsanlage, dem Nachwascher, die C₄-Acetylene ebenfalls durch selektive Gegenstromwäsche mit NMP entfernt. Die in NMP besser als 1,3-Butadien löslichen Komponenten 1-Butin und Butenin gehen in Lösung und am Kopf des Nachwaschers erhält man sogenanntes Roh-1,3-Butadien, ein Kohlenwasserstoffgemisch mit der eingangs definierten Mindestkonzentration an Wertprodukt 1,3-Butadien, das als Verunreinigungen insbesondere noch 1,2-Butadien, Propin cis-2-Buten sowie C₅-Kohlenwasserstoffe enthält.

Das Sumpfprodukt des Nachwaschers, ein mit C₄-Acetylen und 1,3-Butadien beladenes NMP, wird zum Gegenströmer zurückgepumpt. Die C₄-Acetylene findet man im Gegenströmersumpf wieder, von wo aus sie mit dem teilentgasten NMP-Strom zwecks Vollausgasung zur Ausgaserkolonne gepumpt werden. Die Ausschleusung der C₄-Acetylene aus dem System erfolgt als Seitenabzug der Ausgaserkolonne über eine kleine Wasserwäsche zur Vermeidung von Lösungsmittelverlusten sowie partielle Kondensation mit Kühlwasser.

Die Aufbereitung des beladenen NMP's erfolgt nach Aufheizung und Vorausgasung im Gegenströmersumpf in der bereits genannten Ausgaserkolonne, in der man vollständig entgastes NMP am Sumpf erhält und in der am Kopf ein gasförmiger Kohlenwasserstoffstrom anfällt, der über einen Kompressor in den unteren Bereich des Gegenströmers zurückgeführt wird.

Das aus DE-A 27 24 365 bekannte Verfahren zur Gewinnung von Roh-1,3-Butadien durch Extraktivdestillation aus einem C₄-Schnitt hat insbesondere den Nachteil, daß es eine Extraktivdestillationsanlage mit drei Kolonnen erfordert, wobei die mittlere Kolonne, der Gegenströmer, aus hydrodynamischen Gründen mit einem größeren Durchmesser im Unterteil und einem kleineren Durchmesser im Oberteil und von daher mit einer konstruktionstechnisch aufwändigen Einschnürung zwischen Unterteil und Oberteil versehen sein muß.

Es war dem gegenüber Aufgabe der Erfindung, ein verbessertes, insbesondere wirtschaftlicheres Verfahren zur Gewinnung von Roh-1,3-Butadien durch Extraktivdestillation aus einem C₄-Schnitt sowie eine hierfür geeignete Extraktivdestillationsanlage zur Verfügung zu stellen.

Die Aufgabe wird gelöst durch ein Verfahren zur Gewinnung von 1,3-Roh-Butadien durch Extraktivdestillation aus einem C₄-Schnitt mit einem selektiven Lösungsmittel in einer Trennwandkolonne, in der eine Trennwand in Kolonnenlängsrichtung unter Ausbildung eines ersten Teilbereichs , eines zweiten Teilbereichs und eines unteren gemeinsamen Kolonnenbereichs angeordnet ist sowie mit einer vorgeschalteten Extraktivwaschkolonne.

Durch die Erfindung wird somit ein Verfahren zur Gewinnung von Roh-1,3-Butadien durch Extraktivdestillation sowie eine hierfür geeignete Extraktivdestillationsanlage zur Verfügung gestellt, wonach lediglich 2 Kolonnen benötigt werden, die darüber hinaus einen konstanten Durchmesser über die gesamte Kolonnenhöhe und somit keine Verjüngung aufweisen.

Der vorliegend als Ausgangsgemisch einzusetzende sogenannte C₄-Schnitt ist ein Gemisch von Kohlenwasserstoffen mit überwiegend vier Kohlenstoffatomen pro Molekül. C₄-Schnitte werden beispielsweise bei der Herstellung von Ethylen und/oder Propylen durch thermisches Spalten einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, n-Buten, Isobuten, 1,3-Butadien, daneben geringe Mengen an C₃- und C₅-Kohlenwasserstoffen, sowie Butine, insbesondere 1-Butin (Ethylacetylen) und Butenin(Vinylacetylen). Dabei beträgt der 1,3-Butadiengehalt im allgemeinen 10 bis 80 Gew.-%, vorzugsweise 20 bis 70 Gew.-%, insbesondere 30 bis 60 Gew.-%, während der Gehalt an Vinylacetylen und Ethylacetylen im allgemeinen 5 Gew.-% nicht übersteigt.

Ein typischer C₄-Schnitt weist die folgende Zusammensetzung in Gewichtsprozenten auf:

| | |
|---|---|
| Propan | 0 - 0,5 |
| Propen | 0 - 0,5 |
| Propadien | 0 - 0,5 |
| Propin | 0 - 0,5 |
| n-Butan | 3 - 10 |
| i-B utan | 1 - 3 |
| 1-Buten | 10 - 20 |
| i-Buten | 10 - 30 |
| trans-2-Buten | 2 - 8 |
| cis-2-Buten | 2 - 6 |
| 1,3-Butadien | 30 - 60 |
| 1,2-Butadien | 0,1-1 |
| Ethylacetylen | 0,1 - 2 |
| Vinylacetylen | 0,1- 3 |
| C₅-Kohlenwasserstoffe | 0 - 0,5 |

Für die eingangs bereits definierte Extraktivdestillation kommen für das vorliegende Trennproblem, der Gewinnung von 1,3-Butadien aus dem C₄-Schnitt, als Extraktionsmittel, d.h. als selektive Lösungsmittel, generell Substanzen oder Gemische in Frage, die einen höheren Siedepunkt als das aufzutrennende Gemisch sowie eine größere Affinität zu konjugierten Doppelbindungen und Dreifachbindungen als zu einfachen Doppelbindungen sowie Einfachbindungen aufweisen, bevorzugt dipolare, besonders bevorzugt dipolar-aprotische Lösungsmittel. Aus apparatetechnischen Gründen werden wenig oder nicht korrosive Substanzen bevorzugt.

Geeignete selektive Lösungsmittel für das erfindungsgemäße Verfahren sind zum Beispiel Butyrolacton, Nitrile wie Acetonitril, Propionitril, Methoxypropioninitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im allgemeinen werden N-alkylsubstituierte niedere aliphatische Säureamide oder N-alkylsubstituierte cyclische Säureamide verwendet. Besonders vorteilhaft sind Dimethylformamid und insbesondere

N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist N-Methylpyrrolidon, vorliegend abgekürzt als NMP bezeichnet, bevorzugt in wäßriger Lösung, insbesondere mit 7 bis 10 Gew.-% Wasser, besonders bevorzugt mit 8,3 Gew.-% Wasser.

Erfindungsgemäß wird das Verfahren in einer Trennwandkolonne durchgeführt, in der eine Trennwand in Kolonnenlängsrichtung und Ausbildung eines ersten Teilbereichs, eines zweiten Teilbereichs und eines unteren gemeinsamen Kolonnenbereichs angeordnet ist und die mit einer vorgeschalteten Extraktivwaschkolonne verbunden ist.

Trennwandkolonnen werden in bekannter Weise für komplexere Trennaufgaben, in der Regel für Gemische von mindestens drei Komponenten, wobei die Einzelkomponenten jeweils in reiner Form erhalten werden sollen, eingesetzt. Sie weisen eine Trennwand auf, d.h. in der Regel ein in Kolonnenlängsrichtung ausgerichtetes ebenes Blech, das eine Quervermischung der Flüssigkeits- und Brüdenströme in Teilbereichen der Kolonne verhindert.

Vorliegend wird eine besonders ausgestaltete Trennwandkolonne eingesetzt, deren Trennwand bis am obersten Punkt der Kolonne durchgezogen ist und somit eine Vermischung von Flüssigkeits- und Brüdenströmen lediglich im unteren gemeinsamen Kolonnenbereich gestattet. Der sogenannte erste und zweite Teilbereich sind durch die Trennwand voneinander getrennt.

Die Extraktivwaschkolonne ist eine Gegenstromwaschkolonne und entspricht im wesentlichen dem aus dem Stand der Technik bekannten Hauptwascher. Die Extraktivwaschkolonne hat jedoch, bei vergleichbarer Kapazität mit der aus dem Stand der Technik bekannten Anlage, eine geringere Bauhöhe als der Hauptwascher, da ein Teil der Trennaufgabe des Hauptwaschers nunmehr vom oberen Bereich des ersten Teilbereichs der Trennwandkolonne übernommen wird.

Nach einer bevorzugten Verfahrensführung wird
- der C₄-Schnitt dem ersten Teilbereich, bevorzugt in dessen mittleren Bereich, zugeführt,
- der Kopfstrom aus dem ersten Teilbereich der Trennwandkolonne der Extraktivwaschkolonne, in deren unteren Bereich, zugeführt,
- in der Extraktivwaschkolonne eine Gegenstromextraktion durch Beaufschlagung mit einem ersten Teilstrom des selektiven Lösungsmittels im oberen Bereich der Extraktivwaschkolonne durchgeführt,
- die im selektiven Lösungsmittel weniger als 1,3-Butadien löslichen Komponenten des C₄-Schnittes werden über Kopf der Extraktivwaschkolonne abgezogen,
- der Sumpfstrom wird aus der Extraktivwaschkolonne in den oberen Bereich des ersten Teilbereichs der Trennwandkolonne zurückgeführt,
- der Trennwandkolonne wird im mittleren Bereich des zweiten Teilbereichs ein zweiter Teilstrom des selektiven Lösungsmittels zugeführt,
- aus dem Sumpf der Trennwandkolonne wird mit den neben 1,3-Butadien im selektiven Lösungsmittel besser als 1,3-Butadien löslichen Komponenten des C₄-Schnittes beladenes selektives Lösungsmittel abgezogen und
- das Kopfprodukt wird aus dem zweiten Teilbereich der Trennwandkolonne als 1,3-Rohbutadien abgezogen.
   Bevorzugt wird somit der aufzutrennende C₄-Schnitt dem ersten Teilbereich der Trennwandkolonne, besonders bevorzugt in dessen mittleren Bereich, zugeführt;
   der Kopfstrom aus dem ersten Teilbereich der Trennwandkolonne wird der vorgeschalteten Extraktivwaschkolonne in deren unteren Bereich zugeführt,
   in der Extraktivwaschkolonne eine Gegenstromextraktion durch Beaufschlagung mit einem ersten Teilstrom des selektiven Lösungsmittels im oberen Bereich der Extraktivwaschkolonne durchgeführt,
   die im selektiven Lösungsmittel weniger als 1,3-Butadien-löslichen Komponenten des C₄-Schnittes über Kopf der Extraktivwaschkolonne abgezogen, besonders bevorzugt in einem Kondensator am Kopf der Extraktivwaschkolonne kondensiert und teilweise als Rücklauf erneut auf die Extraktivwaschkolonne aufgegeben, im übrigen als ein überwiegend Butane und Butene enthaltendes Nebenprodukt abgezogen.

Durch die Rückführung des Sumpfstroms der Extraktivwaschkolonne, d.h. einem Strom, der neben dem selektiven Lösungsmittel 1,3-Butadien sowie die besser als 1,3-Butadien im selektiven Lösungsmittel löslichen Komponenten des C₄-Schnitts enthält, in den oberen Bereich des ersten Teilbereichs der Trennwandkolonne kann durch den Stoffaustausch zwischen diesem Strom und dem dampfförmig aufgegebenen C₄-Schnitt im oberen Bereich des ersten Teilbereichs der Trennwandkolonne eine Gegenstromextraktion unter Ausschleusung der im selektiven Lösungsmitttel weniger als das 1,3-Butadienlöslichen Komponenten am Kopf des ersten Teilbereichs der Trennwandkolonne erfolgen.

Am unteren Ende der Trennwand fällt ein dampfförmiger Strom an, der neben 1,3-Butadien die im selektiven Lösungsmittel besser als 1,3-Butadien-löslichen Komponenten des C₄-Schnitts, insbesondere C₄-Acetylene, enthält. Diese werden aus dem aufsteigenden dampfförmigen Strom im Gegenstrom mit einem zweiten Teilstrom des selektiven Lösungsmittels, das im mittleren Bereich des zweiten Teilbereichs der Trennwandkolonne aufgegeben wird, ausgewaschen. Das dampfförmige Kopfprodukt aus dem zweiten Teilbereich der Trennwandkolonne wird abgezogen, wobei es bevorzugt in einem Kondensator am Kolonnenkopf kondensiert, ein Teilstrom des kondensierten Kopfstromes zurück auf den Teilbereich B der Trennwandkolonne als Rücklauf gegeben und der kondensierte Kopfstrom wird im übrigen als Roh-1,3-Butadien abgezogen wird.

Der untere gemeinsame Kolonnenbereich der Trennwandkolonne entspricht verfahrenstechnisch dem Unterteil des Gegenströmers der aus dem Stand der Technik bekannten Extraktivdestillationsanlage. Darin erfolgt, analog dem entsprechenden Apparateteil aus dem bekannten Verfahren, eine Vorausgasung der im selektiven Lösungsmittel gelösten Kohlenwasserstoffe, deren Rückführung in den der Verlängerung des Hauptwaschers entsprechenden ersten Teilbereichs der Trennwandkolonne und das Abziehen des teilbeladenen Lösungsmittels aus dem Kolonnensumpf zwecks vollständiger Entgasung zur Ausgaserkolonne.

In einer bevorzugten Verfahrensvariante wird der Brüdenstrom am unteren Ende der Trennwand der Trennwandkolonne durch geeignete Maßnahmen dergestalt aufgetrennt, daß der Teilstrom, der in den ersten Teilbereich der Trennwandkolonne geleitet wird, größer ist als der Teilstrom, der in den zweiten Teilbereich der Trennwandkolonne geleitet wird. Durch die Regelung der Aufteilung des Brüdenstroms am unteren Ende der Trennwand kann in einfacher und zuverlässiger Weise die geforderte Produktspezifikation des am Kopf des zweiten Teilbereichs der Trennwandkolonne abgezogenen Roh-1,3-Butadien-Stroms gewährleistet werden.

Besonders bevorzugt kann eine derartige ungleiche Auftrennung des Brüdenstroms am unteren Ende der Trennwand dadurch erreicht werden, daß die Trennwand außermittig angeordnet wird, und zwar der Gestalt, daß der zweite Teilbereich kleiner ist gegenüber dem ersten Teilbereich der Trennwandkolonne.

Besonders bevorzugt wird die Trennwand außermittig der Gestalt angeordnet, daß das Querschnittsverhältnis des ersten Teilbereichs zum zweiten Teilbereich im Bereich von 8:1 bis 1,5:1, insbesondere bei 2,3:1 liegt.

Alternativ oder zusätzlich zur außermittigen Anordnung der Trennwand kann der Brüdenstrom am unteren Ende der Trennwand durch weitere Maßnahmen, beispielsweise Klappen oder Leitbleche im gewünschten Verhältnis auf die beiden Teilbereiche der Trennwandkolonne aufgeteilt werden.

Eine weitere zusätzliche oder alternative Maßnahme zur Aufteilung des Brüdenstroms am unteren Ende der Trennwand besteht in der Einstellung der Wärrneabfuhrungsleistung des Kondensators am Kopf des zweiten Teilbereichs der Trennwandkolonne.

Eine bevorzugte Verfahrensvariante ist dadurch gekennzeichnet, daß die Drücke am oberen Ende der beiden Teilbereiche der Trennwandkolonne jeweils getrennt regelbar sind. Dadurch kann die erforderliche Produktspezifikation des Roh-1,3-Butadiens sichergestellt werden.

Die Kopfdrücke der beiden Teilbereiche der Trennwandkolonne können bevorzugt jeweils über eine Split-Range-Regelung eingestellt werden. Der Begriff Split-Range-Regelung bezeichnet in bekannter Weise eine Anordnung, wonach der Druckreglerausgang gleichzeitig mit der Inertgas- und der Entlüftungsleitung verbunden ist. Der Ventilstellbereich des Druckreglers ist so geteilt, daß jeweils nur ein Ventil betätigt wird, d.h. entweder es strömt Inertgas zu oder es wird entlüftet. Dadurch können die Inertgasmenge wie auch die mit dem Abluftstrom verbundenen Produktverluste minimiert werden.

Zusätzlich oder alternativ zur Split-Range-Regelung ist es möglich, die Kopfdrücke in den beiden Teilbereichen der Trennwandkolonne jeweils über die Wärmeabfiihrleistung der Kondensatoren am Kopf des zweiten Teilbereichs der Trennwandkolonne bzw. am Kopf der Extraktivwaschkolonne zu regeln.

In einer bevorzugten Verfahrensvariante wird der Kopfdruck im zweiten Teilbereich der Trennwandkolonne größer als im ersten Bereich der Trennwandkolonne eingestellt, insbesondere um 1 - 100 mbar, besonders bevorzugt um 1 - 30 mbar. Durch diese Maßnahme ist es möglich, auf eine fest eingeschweißt oder aufwändig abgedichtete Trennwand zu verzichten und eine kostengünstigere lose Trennwand einzusetzen. Durch das vom zweiten zum ersten Teilbereich der Trennwandkolonne hin gerichtete Druckgefälle können flüssige oder gasförmige Leckargeströme nur in dieser Richtung auftreten, und sind somit unkritisch für die Reinheit des am Kopf des zweiten Teilbereichs abgezogenen Wertprodukts Roh-1,3-Butadien.

Bevorzugt wird der Kopfdruck des zweiten Teilbereichs der Trennwandkolonne auf einen Wert im Bereich von 3 - 7 bar absolut, insbesondere von 4 - 6 bar absolut eingestellt. Dadurch ist es möglich, mit Wasser als Kühlmittel am Kopf der Trennwandkolonne zu kondensieren, aufwändigere Kühlmittel sind nicht erforderlich.

Gegenstand der Erfindung ist auch eine Anlage zur Durchführung des Verfahrens zur Gewinnung von Roh-1,3-Butadien durch Extraktivdestillation aus einem C₄-Schnitt mit einem selektiven Lösungsmittel mit einer Trennwandkolonne, in der eine Trennwand unter Ausbildung eines ersten Teilbereichs, eines zweiten Teilbereichs sowie eines unteren gemeinsamen Kolonnenbereichs in Kolonnenlängsrichtung angeordnet ist sowie mit einer vorgeschalteten Extraktivwaschkolonne.

In einer bevorzugten Ausfiihrungsform enthält die Trennwandkolonne als trennwirksame Einbauten in sämtlichen Kolonnenbereichen, bis auf den oberhalb der Zuführung des zweiten Lösungsmittelteilstroms im zweiten Teilbereich, der mit Böden bestückt ist, Füllkörper oder geordnete Packungen. Alternativ oder zusätzlich enthält die vorgeschaltete Extraktivwaschkolonne bevorzugt oberhalb der Zuführung des ersten Lösungsmittelteilstroms als trennwirksame Einbauten Böden und unterhalb der Zuführung des ersten Lösungsmittelteilstroms Füllkörper oder geordnete Packungen.

Der obere Bereich des zweiten Teilbereichs der Trennwandkolonne, oberhalb der Zuführung des zweiten Lösungsmittelteilstroms muß, wegen der niedrigen Flüssigkeitsbelastung, mit Böden bestückt sein. Dasselbe gilt für den oberen Teilbereich der Extraktivwaschkolonne, oberhalb der Zuführung des ersten Lösungsmittelteilstroms.

Im übrigen sind sowohl in der Trennwandkolonne als auch in der Extraktivwaschkolonne als bevorzugte trennwirksame Einrichtungen Füllkörper oder geordnete Packungen angeordnet.

Aufgrund des höheren Anteils an Komponenten mit verstärkter Polymerisationsneigung und damit erhöhter Verschmutzungsgefahr für die Apparate in den jeweils unteren Bereichen der beiden Teilbereiche der Trennwandkolonne werden dort bevorzugt gröbere Füllkörper oder geordnete Packungen gegenüber den jeweils oberen Bereichen der Teilbereiche der Trennwandkolonne eingesetzt.

Besonders bevorzugt ist eine Anlage mit einer Trennwandkolonne, die im ersten Teilbereich unterhalb der Zuführung des C₄-Schnittes mit einem Füllkörperbett mit 23 theoretischen Stufen und oberhalb der Zuführung des C₄-Schnittes mit einem Füllkörperbett mit 12 theoretischen Stufen, im zweiten Teilbereich oberhalb der Zuführung des zweiten Lösungsmittelteilstroms mit 6 praktischen Böden und unterhalb der Zuführung des zweiten Lösungsmittelteilstroms mit einem Füllkörperbett mit 30 theoretischen Stufen und im unteren gemeinsamen Kolonnenbereich mit einem Füllkörperbett mit 7 theoretischen Stufen und/oder daß die Extraktivwaschkolonne oberhalb der Zuführung des zweiten Lösungsmittelteilstroms in deren oberen Bereich mit den 6 praktischen Böden und einem darunter angeordneten Füllkörperbett mit 15 theoretischen Stufen ausgestattet ist.

Durch die Erfindung wird somit eine Anlage zur Gewinnung von Roh-1,3-Butadien durch Extraktivdestillation aus einem C₄-Schnitt zur Verfügung gestellt, für die gegenüber bekannten Anlagen lediglich zwei gegenüber bislang drei Kolonnen erforderlich sind, wobei diese zwei Kolonnen darüber hinaus mit konstantem Durchmesser über die gesamte Kolonnenhöhe ausgebildet sind. Dadurch sind bei gleicher Kapazität gegenüber einer Anlage aus dem Stand der Technik die Investitionskosten um 10 % niedriger.

Die Erfindung wird im folgenden anhand einer Zeichnung erläutert:
- Figur 1: zeigt das Schema einer Anlage nach der Erfindung mit der schematischen Darstellung der Anordnung der Trennwand in der Trennwandkolonne in Figur 1a
- Figur 2: das Schema eine Anlage nach dem Stand der Technik.

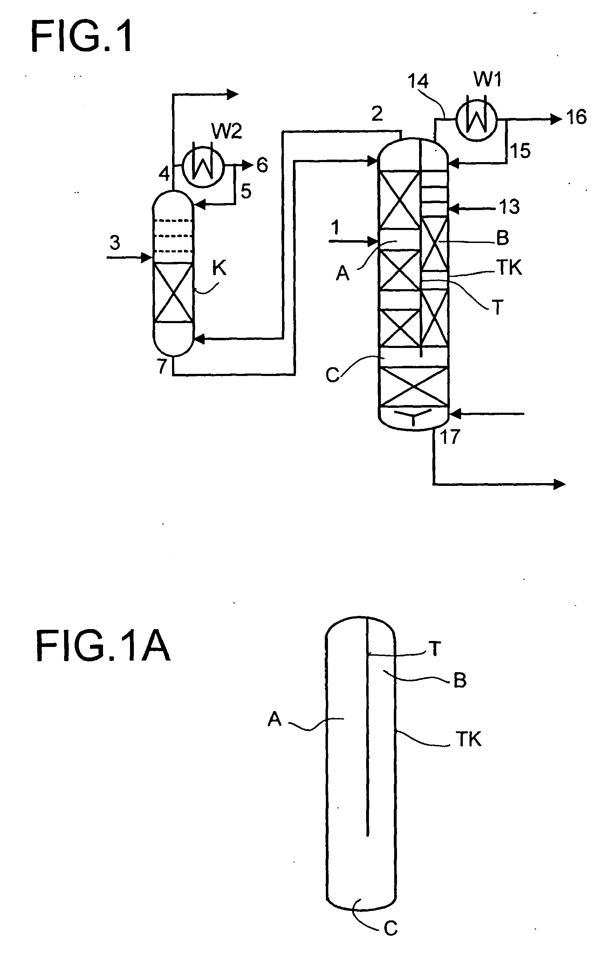

Figur 1 zeigt schematisch eine Anlage nach der Erfindung. In einer Trennwandkolonne TK mit einer in Kolonnenlängsrichtung angeordneten Trennwand T, die die Trennwandkolonne in einen ersten Teilbereich A, einen zweiten Teilbereich B und einen unteren gemeinsamen Kolonnenbereich C aufteilt, wird in den ersten Teilbereich A ein C₄-Schnitt 1 zugeführt. Der Kopfstrom 2 auf dem Teilbereich A wird in den unteren Bereich der vorgeschalteten Extraktivwaschkolonne K geleitet. Die Extraktivwaschkolonne K wird mit einem ersten Lösungsmittelteilstrom 3, in deren oberen Bereich, beaufschlagt, es findet eine Gegenstromextraktion statt, wobei ein Sumpfstrom 7 anfällt, der zurück in den oberen Bereich des Teilbereichs A der Trennwandkolonne TK geführt wird und ein Kopfstrom 4, der in einem Kondensator W2 am Kopf der Extraktivwaschkolonne K kondensiert wird, wobei ein Teilstrom des Kondensats als Strom 5 erneut auf die Extraktivwaschkolonne K aufgegeben und im übrigen als Strom 6 abgezogen wird.

Die Trennwandkolonne TK wird in deren zweiten Teilbereich B mit einem zweiten Lösungsmittelteilstrom 13 beaufschlagt. Aus dem Sumpf der Trennwandkolonne wird ein im unteren gemeinsamen Kolonnenbereich C teilentgaster Strom 17 abgezogen und aus dem zweiten Teilbereich B ein Kopfstrom 14, der im Kondensator W1 kondensiert wird, wobei ein Teilstrom 15 als Rücklauf auf den zweiten Teilbereich B der Trennwandkolonne gegeben und im übrigen als Roh-1,3-Butadien (Strom 16) abgezogen wird.

Zur Verdeutlichung der Anordnung der Trennwand T in der Trennwandkolonne TK und der hierdurch gebildeten Teilbereiche in der Trennwandkolonne TK dient die schematische Darstellung in Figur 1a: Die Trennwand T, die in Längsrichtung der Trennwandkolonne TK angeordnet ist, teilt diese in einen ersten Teilbereich A, einen zweiten Teilbereich B und einen unteren gemeinsamen Kolonnenbereich C.

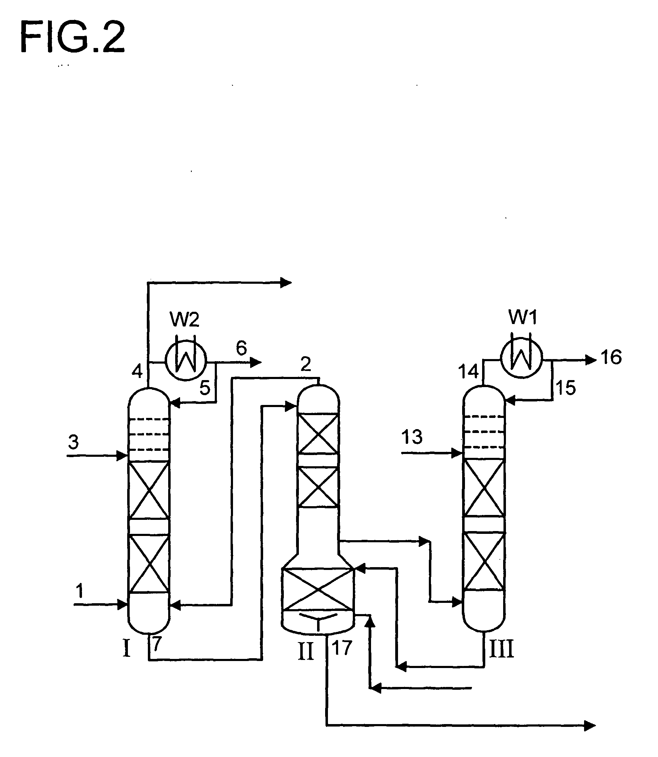

Demgegenüber zeigt Figur 2 das Schema einer Anlage nach dem Stand der Technik. Dabei tragen gleiche Ströme wie in Figur 1 jeweils die gleichen Bezugsziffern. Die drei, die Extraktivdestillationsanlage bildenden Kolonnen wurden mit den Bezugszeichen I bis III bezeichnet.

## Patentansprüche

1. Verfahren zur Gewinnung von Roh-1,3-Butadien durch Extraktivdestillation aus einem C₄-Schnitt mit einem selektiven Lösungsmittel, **dadurch gekennzeichnet, daß** das Verfahren in einer Trennwandkolonne (TK), in der eine Trennwand (T) in Kolonnenlängsrichtung unter Ausbildung eines ersten Teilbereichs (A), eines zweiten Teilbereichs (B) und eines unteren gemeinsamen Kolonnenbereichs (C) angeordnet und der eine Extraktivwaschkolonne (K) vorgeschaltet ist, durchgeführt wird.

2. Verfahren nach Anspruch 1, **dadurch gekennzeichnet, daß**
- der C₄-Schnitt (1) dem ersten Teilbereich (A), bevorzugt in dessen mittleren Bereich, zugeführt wird,
- der Kopfstrom (2) aus dem ersten Teilbereich (A) der Trennwandkolonne (TK) der Extraktivwaschkolonne (K), in deren unteren Bereich, zugeführt wird,
- in der Extraktivwaschkolonne (K) eine Gegenstromextraktion durch Beaufschlagung mit einem ersten Teilstrom des selektiven Lösungsmittels im oberen Bereich der Extraktivwaschkolonne (K) durchgeführt wird,
- die im selektiven Lösungsmittel weniger als 1,3-Butadien löslichen Komponenten des C₄-Schnittes über Kopf der Extraktivwaschkolonne (K) abgezogen (4) werden,
- der Sumpfstrom (5) aus der Extraktivwaschkolonne (K) in den oberen Bereich des ersten Teilbereichs (A) der Trennwandkolonne (TK) zurückgeführt wird,
- der Trennwandkolonne (TK) im mittleren Bereich des zweiten Teilbereichs (B) ein zweiter Teilstrom (13) des selektiven Lösungsmittels zugeführt wird,
- aus dem Sumpf der Trennwandkolonne (TK) mit den neben 1,3-Butadien im selektiven Lösungsmittel besser als 1,3-Butadien löslichen Komponenten des C₄-Schnittes beladenes selektives Lösungsmittel (17) abgezogen wird und
- das Kopfprodukt (14) aus dem zweiten Teilbereich (B) der Trennwandkolonne (TK) als Roh-1,3-Butadien abgezogen wird.

3. Verfahren nach Anspruch 2, **dadurch gekennzeichnet, daß**
- die im selektiven Lösungsmittel weniger als 1,3-Butadien löslichen Komponenten des C₄-Schnittes über Kopf der Extraktivwaschkolonne (K) abgezogen (4), in einem Kondensator (W2) kondensiert, ein Teilstrom des Kondensats (5) als Rücklauf erneut auf die Extraktivwaschkolonne (K) aufgegeben und das Kondensat im übrigen (6) abgeführt wird und/oder daß
- das Kopfprodukt (14) aus dem zweiten Teilbereich (B) der Trennwandkolonne (TK) abgezogen, in einem Kondensator (W1) am Kopf der Trennwandkolonne (TK) kondensiert, ein Teilstrom des kondensierten Kopfstroms zurück auf den Teilbereich (B) der Trennwandkolonne (TK) gegeben und der kondensierte Kopfstrom im übrigen (16) als Roh-1,3-Butadien abgezogen wird.

4. Verfahren nach einem der Ansprüche 1 bis 3, **dadurch gekennzeichnet, daß** der Brüdenstrom am unteren Ende der Trennwand (T) der Trennwandkolonne (TK) durch geeignete Maßnahmen dergestalt aufgetrennt wird, daß der Teilstrom, der in den ersten Teilbereich (A) der Trennwandkolonne (TK) geleitet wird, größer ist als der Teilstrom, der in den zweiten Teilbereich (B) der Trennwandkolonne (TK) geleitet wird.

5. Verfahren nach einem der Ansprüche 1 bis 4, **dadurch gekennzeichnet, daß** es in einer Trennwandkolonne (TK) mit außermittig angeordneter Trennwand (T) durchgeführt wird.

6. Verfahren nach Anspruch 5, **dadurch gekennzeichnet, daß** durch die außermittige Anordnung der Trennwand (T) das Querschnittsverhältnis des ersten Teilbereichs (A) zum zweiten Teilbereich (B) im Bereich von 8:1 bis 1,5:1, insbesondere bei 2,3 : 1 liegt.

7. Verfahren nach einem der Ansprüche 4 bis 6, **dadurch gekennzeichnet, daß** die Aufteilung des Brüdenstroms am unteren Ende der Trennwand (T) über die Einstellung der Wärmeabführungsleistung des Kondensators (W1) am Kopf des zweiten Teilbereichs (B) der Trennwandkolonne (TK) erfolgt.

8. Verfahren nach einem der Ansprüche 1 bis 7, **dadurch gekennzeichnet, daß** die Drücke am oberen Ende der Teilbereiche (A) und (B) jeweils getrennt regelbar sind.

9. Verfahren nach Anspruch 8, **dadurch gekennzeichnet, daß** die Kopfdrücke der beiden Teilbereiche (A) und (B) der Trennwandkolonne (TK) jeweils über eine Split-range-Regelung eingestellt werden.

10. Verfahren nach einem der Ansprüche 1 bis 9, **dadurch gekennzeichnet, daß** die Kopfdrücke in den beiden Teilbereichen (A) und (B) der Trennwandkolonne (TK) jeweils über die Wärmeabführleistung der Kondensatoren (W1) und (W2) geregelt werden.

11. Verfahren nach einem der Ansprüche 1 bis 10, **dadurch gekennzeichnet, daß** der Kopfdruck im Teilbereich (B) der Trennwandkolonne (TK) größer ist als der Kopfdruck im Teilbereich (A) der Trennwandkolonne (TK), insbesondere um 1 bis 100 mbar, besonders bevorzugt um 1 bis 30 mbar.

12. Verfahren nach einem der Ansprüche 1 bis 11, **dadurch gekennzeichnet, daß** der Kopfdruck des Teilbereichs (B) der Trennwandkolonne (TK) auf einen Wert im Bereich von 3 bis 7 bar absolut, insbesondere von 4 bis 6 bar absolut, eingestellt wird.

13. Anlage zur Durchführung des Verfahrens nach einem der Ansprüche 1 bis 12, mit einer Trennwandkolonne (TK), in der Trennwand (T) unter Ausbildung eines ersten Teilbereichs (A), eines zweiten Teilbereichs (B) sowie eines unteren gemeinsamen Kolonnenbereichs (C) in Kolonnenlängsrichtung angeordnet ist, sowie mit einer vorgeschalteten Extraktivwaschkolonne (K).

14. Anlage nach Anspruch 13, **dadurch gekennzeichnet, daß** die Trennwandkolonne (TK) als trennwirksame Einbauten in sämtlichen Kolonnenbereichen, bis auf den Bereich oberhalb der Zuführung des zweiten Lösungsmittelteilstroms (13) im zweiten Teilbereich (B), der mit Böden bestückt ist, Füllkörper oder geordnete Packungen enthält und/oder daß die vorgeschaltete Extraktivwaschkolonne (K) oberhalb der Zuführung des ersten Lösungsmittelteilstroms (3) als trennwirksame Einbauten Böden und unterhalb der Zuführung des ersten Lösungsmittelteilstroms (3) Füllkörper oder geordnete Packungen enthält.

15. Anlage nach Anspruch 13 oder 14, **dadurch gekennzeichnet, daß** im unteren gemeinsamen Kolonnenbereich (C) sowie in den unteren Bereichen der Teilbereiche (A) und (B) der Trennwandkolonne (TK) gröbere Füllkörper oder geordnete Packungen eingesetzt werden gegenüber den oberen Bereichen der Teilbereiche (A) und (B) der Trennwandkolonne (TK).

16. Anlage nach einem der Ansprüche 13 bis 15, **dadurch gekennzeichnet, daß** die Trennwandkolonne (TK) im ersten Teilbereich (A) unterhalb der Zuführung des C₄-Schnittes mit einem Füllkörperbett mit 23 theoretischen Stufen und oberhalb der Zuführung des C₄-Schnittes mit einem Füllkörperbett mit 12 theoretischen Stufen, im zweiten Teilbereich (B) oberhalb der Zuführung des zweiten Lösungsmittelteilstroms (13) mit 6 praktischen Böden und unterhalb der Zuführung des zweiten Lösungsmittelteilstroms (13) mit einem Füllkörperbett mit 30 theoretischen Stufen und im unteren gemeinsamen Kolonnenbereich (C) mit einem Füllkörperbett mit 7 theoretischen Stufen und/oder
daß die Extraktivwaschkolonne (K) oberhalb der Zuführung des ersten Lösungsmittelteilstroms (3) in deren oberen Bereich mit den 6 praktischen Böden und einem darunter angeordneten Füllkörperbett mit 15 theoretischen Stufen ausgestattet ist.

## Claims

1. A process for recovering crude 1, 3-butadiene from a C₄ fraction by extractive distillation using a selective solvent, which is carried out in a dividing wall column (TK) in which a dividing wall (T) is arranged in the longitudinal direction of the column to form a first subregion (A), a second subregion (B) and a lower common column region (C) and which is preceded by an extractive scrubbing column (K).

2. The process according to claim 1, wherein
- the C₄ fraction (1) is fed to the first subregion (A), preferably in its middle region,
- the stream (2) taken off at the top from the first subregion (A) of the dividing wall column (TK) is fed to the extractive scrubbing column (K) in its upper region,
- a countercurrent extraction is carried out in the extractive scrubbing column (K) by treatment with a first substream of the selective solvent in the upper region of the extractive scrubbing column (K),
- the components of the C₄ fraction which are less soluble than 1,3-butadiene in the selective solvent are taken off (4) at the top of the extractive scrubbing column (K),
- the bottom stream (5) from the extractive scrubbing column (K) is recirculated to the upper region of the first subregion (A) of the dividing wall column (TK),
- a second substream (13) of the selective solvent is fed to the dividing wall column (TK) in the middle region of the second subregion (B),
- selective solvent (17) laden with 1,3-butadiene together with components of the C₄ fraction which are more soluble than 1,3-butadiene in the selective solvent is taken off from the bottom of the dividing wall column (TK) and
- the product (14) taken off at the top from the second subregion (B) of the dividing wall column (TK) as crude 1,3-butadiene.

3. The process according to claim 2, wherein
- the components of the C₄ fraction which are less soluble than 1,3-butadiene in the selective solvent are taken off (4) at the top of the extractive scrubbing column (K) and condensed in a condenser (W2), a substream of the condensate (5) is returned as runback to the extractive scrubbing column (K) and the remainder of the condensate (6) is discharged, and/or
- the product (14) taken off at the top from the second subregion (B) of the dividing wall column (TK) is condensed in a condenser (W1) at the top of the dividing wall column (TK), a substream of the condensed top product is returned to the subregion (B) of the dividing wall column (TK) and the remainder of the condensed top stream (16) is taken off as crude 1,3-butadiene.

4. The process according to any of claims 1 to 3, wherein the stream of vapor is divided at the lower end of the dividing wall (T) of the dividing wall column (TK) by means of suitable measures so that the substream conveyed into the first subregion (A) of the dividing wall column (TK) is larger than the substream conveyed into the second subregion (B) of the dividing wall column (TK) .

5. The process according to any of claims 1 to 4 carried out in a dividing wall column (TK) having a noncentrally arranged dividing wall (T).

6. The process according to claim 5, wherein, due to the noncentral arrangement of the dividing wall (T), the cross-sectional ratio of the first subregion (A) to the second subregion (B) is in the range from 8:1 to 1.5:1, in particular 2.3:1.

7. The process according to any of claims 4 to 6, wherein the division of the stream of vapor at the lower end of the dividing wall (T) is carried out by setting the heat removal power of the condenser (W1) at the top of the second subregion (B) of the dividing wall column (TK).

8. The process according to any of claims 1 to 7, wherein the pressures at the upper end of the subregions (A) and (B) can each be regulated separately.

9. The process according to claim 8, wherein the pressures at the top of the two subregions (A) and (B) of the dividing wall column (TK) are each set via a split-range control.

10. The process according to any of claims 1 to 9, wherein the pressures at the top of the two subregions (A) and (B) of the dividing wall column (TK) are each regulated via the heat removal power of the condensers (W1) and (W2).

11. The process according to any of claims 1 to 10, wherein the pressure at the top of the subregion (B) of the dividing wall column (TK) is greater than the pressure at the top of the subregion (A) of the dividing wall column (TK), in particular by from 1 to 100 mbar, particularly preferably by from 1 to 30 mbar.

12. The process according to any of claims 1 to 11, wherein the pressure at the top of the subregion (B) of the dividing wall column (TK) is set to a value in the range from 3 to 7 bar absolute, in particular from 4 to 6 bar absolute.

13. An apparatus for carrying out the process according to any of claims 1 to 12 comprising a dividing wall column (TK) in which a dividing wall (T) is arranged in the longitudinal direction of the column to form a first subregion (A), a second subregion (B) and a lower common column region (C) and an upstream extractive scrubbing column (K).

14. The apparatus according to claim 13, wherein the dividing wall column (TK) comprises random packing elements or ordered packing as separation-active internals in all regions of the column except for the region above the inlet for the second solvent substream (13) in the second subregion (B) which is provided with trays, and/or the upstream extractive scrubbing column (K) comprises trays as separation-active internals above the inlet for the first solvent substream (3) and comprises random packing elements or ordered packing below the inlet for the first solvent substream (3).

15. The apparatus according to claim 13 or 14, wherein coarser random packing elements or ordered packing are used in the lower common column region (C) and in the lower regions of the subregions (A) and (B) of the dividing wall column (TK) compared to the upper regions of the subregions (A) and (B) of the dividing wall column (TK).

16. The apparatus according to any of claims 13 to 15, wherein the dividing wall column (TK) is equipped with a bed of random packing elements having 23 theoretical plates in the first subregion (A) below the inlet for the C₄ fraction and with a bed of random packing elements having 12 theoretical plates above the inlet for the C₄ fraction, with 6 practical trays in the second subregion (B) above the inlet for the second solvent substream (13) and with a bed of random packing elements having 30 theoretical plates below the inlet for the second solvent substream (13) and with a bed of random packing elements having 7 theoretical plates in the lower common column region (C), and/or
the extractive scrubbing column (K) is equipped with 6 practical trays in its upper region above the inlet for the first solvent substream (3) and with a bed of random packing elements having 15 theoretical plates located underneath.

## Revendications

1. Procédé d'obtention de 1,3-butadiène brut par distillation extractive d'une coupe de C₄ avec un solvant sélectif, **caractérisé en ce que** le procédé est effectué dans une colonne à paroi de séparation (TK) dans laquelle une paroi de séparation (T) est agencée dans le sens longitudinal de la colonne avec formation d'une première zone partielle (A), d'une deuxième zone partielle (B) et d'une zone de colonne inférieure commune (C) et en amont de laquelle se trouve une tour de lavage par extraction (K).

2. Procédé selon la revendication 1, **caractérisé en ce que** :
- la coupe de C₄ (1) est conduite à la première zone partielle (A), de préférence dans la zone centrale de celle-ci,
- le courant de tête (2) provenant de la première zone partielle (A) de la colonne à paroi de séparation (TK) est conduit à la tour de lavage par extraction (K), dans la zone inférieure de celle-ci,
- dans la tour de lavage par extraction (K) se produit une extraction à contre-courant par injection d'un premier courant partiel du solvant sélectif dans la zone supérieure de la tour de lavage par extraction (K),
- les composants de la coupe de C₄ qui se dissolvent moins bien dans le solvant sélectif que le 1,3-butadiène sont soutirés (4) par la tête de la tour de lavage par extraction (K),
- le courant de fond (5) est reconduit depuis la tour de lavage par extraction (K) dans la zone supérieure de la première zone partielle (A) de la colonne à paroi de séparation (TK),
- un deuxième courant partiel (13) du solvant sélectif est conduit à la colonne à paroi de séparation (TK) dans la zone centrale de la deuxième zone partielle (B),
- on soutire du fond de la colonne à paroi de séparation (TK), avec les composants de la coupe de C₄ qui à côté du 1,3-butadiène se dissolvent dans le solvant sélectif mieux que le 1,3-butadiène, le solvant sélectif chargé (17), et
- le produit de tête (14) est soutiré de la deuxième zone partielle (B) de la colonne à paroi de séparation (TK) sous forme de 1,3-butadiène brut.

3. Procédé selon la revendication 2, **caractérisé en ce que** :
- les composants de la coupe de C₄ qui se dissolvent dans le solvant sélectif moins bien que le 1,3-butadiène sont soutirés (4) par la tête de la tour de lavage par extraction (K), sont condensés dans un condenseur (W2), un courant partiel du condensat (5) est renvoyé comme reflux à la tour de lavage par extraction (K) et le condensat est, pour le reste (6), évacué et/ou **en ce que** :
- le produit de tête (14) de la deuxième zone partielle (3) de la colonne à paroi de séparation (TK) est soutiré, condensé dans un condenseur (W1) à la tête de la colonne à paroi de séparation (TK), un courant partiel du courant de tête condensé est renvoyé à la zone partielle (B) de la colonne à paroi de séparation (TK) et le courant de tête condensé est, pour le reste (16), soutiré sous forme de 1,3-butadiène brut.

4. Procédé selon l'une des revendications 1 à 3, **caractérisé en ce que** le courant de vapeur à l'extrémité inférieure de la paroi de séparation (T) de la colonne à paroi de séparation (TK) est séparé par des mesures appropriées de telle façon que le courant partiel conduit dans la première zone partielle (A) de la colonne à paroi de séparation (TK) est plus grand que le courant partiel qui est conduit dans la deuxième zone partielle (B) de la colonne à paroi de séparation (TK).

5. Procédé selon l'une des revendications 1 à 4,
**caractérisé en ce qu'**il est réalisé dans une colonne à paroi de séparation (TK) avec paroi de séparation (T) agencée de manière excentrée.

6. Procédé selon la revendication 5, **caractérisé en ce que** l'agencement excentrique de la paroi de séparation (T) fait que le rapport de coupe transversale de la première zone partielle (A) à la deuxième zone partielle (B) se situe dans l'intervalle entre 8:1 et 1,5:1, en particulier à 2,3:1.

7. Procédé selon l'une des revendications 4 à 6, **caractérisé en ce que** la répartition du courant de vapeur à l'extrémité inférieure de la paroi de séparation (T) s'effectue par réglage de la capacité d'évacuation de la chaleur du condenseur (W1) à la tête de la deuxième zone partielle (B) de la colonne à paroi de séparation (TK).

8. Procédé selon l'une des revendications 1 à 7, **caractérisé en ce que** les pressions à l'extrémité supérieure des zones partielles (A) et (B) sont toutes individuellement réglables.

9. Procédé selon la revendication 8, **caractérisé en ce que** les pressions de tête des deux zones partielles (A) et (B) de la colonne à paroi de séparation (TK) sont réglées chaque fois par régulation split-range.

10. Procédé selon l'une des revendications 1 à 9, **caractérisé en ce que** les pressions de tête dans les deux zones partielles (A) et (B) de la colonne à paroi de séparation (TK) sont chaque fois réglées par la capacité d'évacuation de la chaleur des condenseurs (W1) et (W2).

11. Procédé selon l'une des revendications 1 à 10, **caractérisé en ce que** la pression de tête dans la zone partielle (B) de la colonne à paroi de séparation (TK) est plus élevée que la pression de tête dans la zone partielle (A) de la colonne à paroi de séparation (TK), en particulier de 1 à 100 mbar, de manière particulièrement préférée de 1 à 30 mbar.

12. Procédé selon l'une des revendications 1 à 11, **caractérisé en ce que** la pression de tête de la zone partielle (B) de la colonne à paroi de séparation (TK) est réglée à une valeur comprise dans l'intervalle entre 3 et 7 bars absolus, en particulier entre 4 et 6 bars absolus.

13. Installation d'exécution du procédé selon l'une des revendications 1 à 12, avec une colonne à paroi de séparation (TK), dans laquelle est agencée une paroi de séparation (T) avec formation d'une première zone partielle (A), d'une deuxième zone partielle (B) ainsi que d'une zone de colonne inférieure commune (C) dans la direction longitudinale de la colonne, ainsi qu'avec une tour de lavage par extraction (K) prévue en amont.

14. Installation selon la revendication 13, **caractérisée en ce que** la colonne à paroi de séparation (TK) contient en tant qu'éléments encastrés servant à la séparation dans toutes les zones de la colonne, jusqu'à la zone au-dessus de l'amenée du deuxième courant partiel de solvant (13) dans la deuxième zone partielle (B), laquelle est équipée de plateaux, des charges ou des garnitures agencées et/ou **en ce que** la tour de lavage par extraction en amont (K) contient, en tant qu'éléments encastrés servant à la séparation, au-dessus de l'arrivée du premier courant partiel de solvant (3), des plateaux et en dessous de l'arrivée du premier courant partiel de solvant (3), des charges ou des garnitures agencées.

15. Installation selon la revendication 13 ou 14, **caractérisée en ce que** dans la zone de colonne inférieure commune (C) ainsi que dans les zones inférieures des zones partielles (A) et (B) de la colonne à paroi de séparation (TK), des charges plus grossières ou des garnitures agencées sont insérées en face des zones supérieures des zones partielles (A) et (B) de la colonne à paroi de séparation (TK) .

16. Installation selon l'une des revendications 13 à 15, **caractérisée en ce que** la colonne à paroi de séparation (TK) est équipée, dans la première zone partielle (A) en dessous de l'alimentation en coupe de C₄, d'un lit de matière de charge avec 23 plateaux théoriques et au-dessus de l'alimentation en coupe de C₄, d'un lit de matière de charge avec 12 plateaux théoriques, dans la deuxième zone partielle (B) au-dessus de l'alimentation en deuxième courant partiel de solvant (13), de 6 plateaux pratiques et en dessous de l'alimentation en deuxième courant partiel de solvant (13) d'un lit de matière de charge avec 30 plateaux théoriques et, dans la zone de colonne inférieure commune (C), d'un lit de matière de charge avec 7 plateaux théoriques et/ou
**en ce que** la tour de lavage par extraction (K) au-dessus de l'arrivée du premier courant partiel de solvant (3) dans la zone supérieure de celle-ci, est équipée des 6 plateaux théoriques et d'un lit de matière de charge agencé en dessous de ceux-ci avec 15 plateaux théoriques.
